# EUROPÄISCHE PATENTANMELDUNG

(11) **EP 4 066 815 A1**
(43) Veröffentlichungstag der Anmeldung: **05.10.2022**
(21) Anmeldenummer: 21165879.4
(22) Anmeldetag: 30.03.2021
(51) Int. Cl.: A61K 8/9789, A61K 8/98, A61Q 11/00, A61K 8/9783, A61K 8/9794

(54) **ZAHNREINIGUNGSMITTEL**

(71) Anmelder: Stallinger, Martin, 94110 Wegscheid (DE)
(72) Erfinder: Stallinger, Martin, 94110 Wegscheid (DE)
(74) Vertreter: advotec.

(57) **Zusammenfassung**

Die Erfindung betrifft ein Zahnreinigungsmittel, insbesondere Zahnpasta, Zahngel oder dergleichen, enthaltend Wasser, mindestens einen Putzkörper sowie mindestens ein Feuchthaltemittel, dadurch gekennzeichnet, dass es Propolis und/oder mindestens einen pflanzlichen Zusatz, ausgewählt aus der Gruppe von Kurkuma, Jiaogulan, Ginseng, Aloe, Lepidium, Sambung Nyawa, Goji Beere, Schisandra, Chinesische Yamswurzel, Fo-Tieng, Ginkgo, Chinesische Tragant, Dong-Ling-Cao, Gao Ben, Süßholz, Mo-Han-Lian, Niu Xi, Chinesischer Molchschwanz, Moringa und Ashwagandha enthält.

## Beschreibung

Die Erfindung betrifft ein Zahnreinigungsmittel, insbesondere Zahnpasta, Zahngel oder dergleichen, enthaltend Wasser, Putzkörper sowie mindestens ein Feuchthaltemittel.

Derartige Zahnreinigungsmittel sind aus dem Stand der Technik bekannt und dienen in erster Linie zum Reinigen der Zähne. Eine zusätzliche Beimengung von bestimmten Zusatzstoffen, wie beispielsweise ätherischen Ölen oder dergleichen kann zu einem frischen Atem nach dem Zähneputzen beitragen. Um den Zahnschmelz zu stärken, enthalten manche der bekannten Zahnreinigungsmittel Mineralien. So ist beispielsweise aus DE 3889895 T2 eine Zahnpasta mit Tonerde bekannt, die aus einer homogenen Mischung aus Tonerde, Glycerin und Wasser besteht und außerdem Meersalz enthalten kann. Weitere für den Benutzer vorteilhafte Bestandteile sind in den bekannten Zahnreinigungsmitteln nicht enthalten.

Der vorliegenden Erfindung liegt die Aufgabe zugrunde, ein Zahnreinigungsmittel zur Verfügung zu stellen, welches eine gute Reinigungswirkung aufweist und gleichzeitig eine positive Wirkung auf den Körper des Benutzers hat.

Diese Aufgabe wird durch ein Zahnreinigungsmittel der eingangs genannten Art gelöst, welches dadurch gekennzeichnet ist, dass es Propolis und/oder mindestens einen pflanzlichen Zusatz, ausgewählt aus der Gruppe von Kurkuma, Jiaogulan, Ginseng, Aloe, Lepidium, Sambung Nyawa, Goji Beere, Schisandra, Chinesische Yamswurzel, Fo-Tieng, Ginkgo, Chinesische Tragant, Dong-Ling-Cao, Gao Ben, Süßholz, Mo-Han-Lian, Niu Xi, Chinesischer Molchschwanz, Moringa und Ashwagandha, enthält.

Die im erfindungsgemäßen Zahnreinigungsmittel enthaltenen pflanzlichen Zusätze bzw. Propolis werden allesamt beim Putzen der Zähne von der Mundschleimhaut aufgenommen und entfalten dann ihre positiven Wirkungen.

Propolis, auch Stopfwachs, Bienenharz, Bienenleim, Bienenkitharz, Kitharz oder Kitwachs genannt, ist eine von Bienen hergestellte harzartige Masse mit antibiotischer, antiviraler und antimykotischer Wirkung. Propolis ist ein Gemisch aus vielen unterschiedlichen Stoffen, deren Zusammensetzung stark variieren kann. Der Grundstoff wird von Honigbienen als harzige Substanz an Knospen und teilweise an Wunden verschiedener Bäume gesammelt (ca. 55% Naturharz und Pollenbalsam). Weiter verarbeitet, mit etwa 30% Wachs, etwa 5% Pollenanteilen, etwa 10% ätherischen Ölen aus den Blütenknospen und Speichelsekret angereichert, handelt es sich um ein klebriges Material, das oft noch mit Bienenteilen und kleinsten Holzstücken verunreinigt ist. Durch die antibiotische und antivirale Wirkung kann beispielsweise Zahnfleischentzündungen und anderen bakteriellen und/oder viralen Erkrankungen im Mundbereich vorgebeugt bzw. können solche Erkrankungen behandelt werden.

Kurkuma wirkt beispielsweise Blähungen und Völlegefühl entgegen und zeigt antientzündliche Effekte. Ferner konnte nachgewiesen werden, dass Kurkuma Schmerzen und Gelenksteifigkeit mindern kann. Durch die Tatsache, dass beispielsweise das gelbgefärbte Kurkuma in einem herkömmlichen Grundmaterial einer Zahncreme enthalten ist, wird erreicht, dass die gelbe Farbe dieser Pflanze vom Grundmaterial zumindest teilweise neutralisiert wird.

Jiaogulan ist in der traditionellen Medizin als das Kraut der Unsterblichkeit bekannt. Bei dieser Pflanze werden in erster Linie die Blätter verwendet. Dieses Pflanzenmaterial hat insbesondere positive Wirkung auf Herz und Kreislauf und wirkt gegen Stoffwechselschwäche sowie Blut- und Gefäßerkrankungen.

Ginseng, von welchem die Wurzel verwendet wird, wirkt bei nachlassender Leistungs- und Konzentrationsfähigkeit sowie in der Genesungsphase. Zudem wurden positive Wirkungen bei Rheuma gefunden.

Aloe, insbesondere Aloe Vera wirkt gegen Verstopfung und ist hervorragend für die Behandlung von Zahnfleischentzündungen geeignet.

Lepidium, von welchem in erster Linie die Wurzeln verwendet werden, steigert die Spermienproduktion und fördert die Durchblutung des Beckengewebes. Zudem kurbelt es die Testosteron- bzw. Östrogenbildung an.

Sambung Nyawa wirkt entgiftend und blutreinigend. Bei dieser Pflanze werden in erster Linie die Blätter zur Herstellung des erfindungsgemäßen Zahnreinigungsmittels verwendet.

Goji Beeren wirken gegen unreine Haut und stärken die Sehkraft.

Schisandrafrüchte wirken wundheilend und eignen sich daher besonders gut für die Behandlung von Zahnfleischbluten.

Die Yamswurzel (wissenschaftlich: *Dioscorea*) wirkt u. a. gegen Menstruationsbeschwerden.

Fo-Tieng (Polygonum multiflorum) wirkt senkend auf den Cholesterinspiegel sowie den Blutzuckerspiegel und hat eine blutreinigende Wirkung.

Gingko, insbesondere Gingko-Extrakt wirkt prophylaktisch gegen Demenz sowie gegen Durchblutungsstörungen.

Beim Chenesischen Tragant (Astragalus mongholicus) werden insbesondere die reif geernteten Wurzeln verwendet. Dieser pflanzliche Zusatz hat sich als wirksam bei der Behandlung von Heuschnupfen erwiesen. Durch die schnelle Aufnahme über die Mundschleimhaut wird eine schnelle Linderung der Beschwerden erzielt.

Dong-Ling-Cao wirkt entzündungshemmend. Bei dieser Pflanze werden insbesondere die Blüten und Blätter für das erfindungsgemäße Zahnreinigungsmittel verarbeitet.

Gao Ben (auch chinesischer Liebstöckel) wirkt schmerzlindernd. Von dieser Pflanze werden insbesondere die Wurzeln verwendet.

Süßholz (Glycyrrhiza glabra), insbesondere chinesisches Süßholz (Glycyrrhiza uralensis) wirkt gegen Beschwerden bei grippalen Infekten sowie Magenbeschwerden.

Mo-Han-Lian (Ecliptae herba) wirkt bei Leber- und Nierenbeschwerden.

Niu Xi (Achyranthes-bidentata-Wurzel) tonisiert die Leber und die Nieren und stärkt Knochen und Sehnen. Es wirkt zudem bei Rücken- sowie Gliederschmerzen.

Chinesischer Molchschwanz (Saururus chinensis) wirkt entzündungshemmend und entwässernd. Zudem ist es als Entgiftungsmittel bekannt. Es wirkt auch gegen rheumatoide Arthritis.

Moringa (insbesondere Moringa oleifera) stärkt das Immunsystem und regt die Durchblutung und den Stoffwechsel an.

Ashwagandha wirkt beruhigend und schlaffördernd. Es ist deshalb insbesondere für Zahnreinigungsmittel geeignet, welche vor dem Schlafengehen benutzt werden.

Mit dem erfindungsgemäßen Zahnreinigungsmittel ist es also auf ideale Art und Weise möglich, neben dem herkömmlichen Reinigen der Zähne auch einen therapeutischen oder prophylaktischen Effekt zu erzielen.

In der Regel liegt der pflanzliche Zusatz als Pulver, Essenz und/oder Paste vor.

Mit Vorteil wird der pflanzliche Zusatz gewonnen, indem aus der jeweiligen Pflanze, insbesondere der Wurzel und/oder den Blättern entweder i) durch Trocknen und Pulverisieren des Pflanzenmaterials ein Pulver oder ii) durch Kochen in Wasser ein wässriger Sud oder iii) durch Einlegen in Alkohol ein alkoholischer Auszug oder iiii) durch Verarbeitung des fein zerriebenen Pflanzenmaterials mit mehr oder minder viskosen Flüssigkeiten eine Paste gewonnen wird. Durch diese Art der Gewinnung des pflanzlichen Zusatzes wird erreicht, dass sämtliche therapeutisch wirksamen Inhaltsstoffe im erfindungsgemäßen Zahnreinigungsmittel erhalten bleiben.

In der Regel enthält das erfindungsgemäße Zahnreinigungsmittel als Putzkörper Siliciumoxid, Calciumphosphat, Kaolin, Natriumpolyphosphat und/oder Kreide.

Als Feuchthaltemittel kann das erfindungsgemäße Zahnreinigungsmittel Glycerin, Sorbitol und/oder Mannit enthalten.

Mit Vorteil enthält das erfindungsgemäße Zahnreinigungsmittel Zusatzstoffe, vorzugsweise eine Fluoridquelle, insbesondere in Form von Flussspat, Natriumfluorid, Natriummonoflurphosphat und/oder Kaliumfluorid, Tenside und/oder Aromastoffe. Es ist bekannt, dass FluoridIonen innerhalb weniger Sekunden mit äußeren Schmelzschichten der Zähne biochemisch reagieren können. Fluoride bilden bekanntermaßen mit den Schmelzkristallen Calciumhydroxylfluorapatit.

Mit Vorteil weist das erfindungsgemäße Zahnreinigungsmittel Natriumhydrogencarbonat auf. Dieses kann mit einem Anteil von ca. 10 bis ca. 35 Gew.-%, insbesondere 30 bis 35 Gew.-% in dem erfindungsgemäßen Zahnreinigungsmittel enthalten sein. Natriumhydrogencarbonat hat verschiedene vorteilhafte Wirkungen. So neutralisiert es Säuren unter Bildung von Wasser und Kohlendioxid. Dadurch unterstützt es die natürliche Fähigkeit des Speichels zur Neueinlagerung von Calcium und Phosphaten in den Zahnschmelz. Mit Calcium und Phosphat gesättigter Speichel kann die säurebedingten Mineralverluste im Zahnschmelz reparieren. Azidogene Mikroorganismen finden optimale Lebensbedingungen und vermehren sich in einem angesäuerten Milieu gut. Natriumhydrogencarbonat macht aus dem bakteriell sauren Biotop einen biochemisch neutralen Lebensraum. Dieser erschwert deutlich die Lebensbedingungen für die Vermehrung azidophiler Bakterien. Hohe Konzentrationen von Natriumhydrogencarbonat entwickeln in der Mundhöhle eine hohe osmotische Kraft. Dies trifft besonders für die Zahnzwischenräume und die Zahnfleischtaschen zu. Dort wirkt Natriumhydrogencarbonat auf Bakterien vorübergehend abtötend. Natriumhydrogencarbonat kann auch als Putzkörper fungieren. Die Kristalle dieser Verbindung sind im Vergleich zu konventionellen Putzkörpern deutlich größer. Allerdings sind sie auch wesentlich weicher. Diese "sanften" Kristalle begünstigen eine bessere Entfernung von Plaque. Dieser ist über eine Polysaccharidmatrix mit der Zahnoberfläche verbunden.

Ferner kann das erfindungsgemäße Zahnreinigungsmittel Xanthan Gum aufweisen. Xanthan Gum beeinflusst die Konsistenz, insbesondere die Cremigkeit eines Zahnreinigungsmittels in Form einer Paste.

Mit Vorteil weist das erfindungsgemäße Zahnreinigungsmittel Titanoxid, insbesondere Titandioxid auf. Titandioxid ist ein ungiftiges Weißpigment mit einem hohen Deckvermögen. Insbesondere in Kombination mit farbigen pflanzlichen Zusätzen, wie beispielsweise Kurkuma neutralisiert Titandioxid die Farben derartiger Zusätze, sodass das Zahnreinigungsmittel auch bei Anwesenheit farbiger pflanzlicher Zusatzstoffe ein weißes Erscheinungsbild behält.

Das erfindungsgemäße Zahnreinigungsmittel kann des Weiteren einen Süßstoff, insbesondere Saccharin aufweisen. Ein solcher Süßstoff verleiht dem erfindungsgemäßen Zahnreinigungsmittel einen angenehmen süßlichen Geschmack.

Mit Vorteil weist das erfindungsgemäße Zahnreinigungsmittel Zinkchlorid und/oder Methylparaben auf. Zinkchlorid und Methylparaben haben die vorteilhafte Wirkung, dass sie das Zahnreinigungsmittel vor bakteriellem Befall schützen.

Mit Vorteil enthält das erfindungsgemäße Zahnreinigungsmittel ca. 20 bis ca. 40 Gew.-% Wasser, ca. 10 bis ca. 20 Gew.-% Putzkörper, ca. 10 bis ca. 45 Gew.-% Feuchthaltemittel, ca. 10 Gew.-% Zusatzstoffe und ca. 0,1 bis ca. 20 Gew.-% pflanzlichen Zusatz und/oder Propolis.

Die Erfindung wird nachfolgend anhand eines Ausführungsbeispiels näher erläutert.

### Beispiel:

Zur Herstellung einer erfindungsgemäßen Zahncreme wird eine wässrige Mischung (Paste) aus 50 Gew.-% Wasser, 20 Gew.-% Kieselsäure als Putzkörper, 25 Gew.-% Glycerin und 5 Gew.-% Kurkuma hergestellt. Bedarfsweise kann zusätzlich eine Fluoridquelle eingesetzt werden.

## Patentansprüche

1. Zahnreinigungsmittel, insbesondere Zahnpasta, Zahngel oder dergleichen, enthaltend Wasser, mindestens einen Putzkörper sowie mindestens ein Feuchthaltemittel, **dadurch gekennzeichnet, dass** es Propolis und/oder mindestens einen pflanzlichen Zusatz, ausgewählt aus der Gruppe von Kurkuma, Jiaogulan, Ginseng, Aloe, Lepidium, Sambung Nyawa, Goji Beere, Schisandra, Chinesische Yamswurzel, Fo-Tieng, Ginkgo, Chinesische Tragant, Dong-Ling-Cao, Gao Ben, Süßholz, Mo-Han-Lian, Niu Xi, Chinesischer Molchschwanz, Moringa und Ashwagandha enthält.

2. Zahnreinigungsmittel nach Anspruch 1, **dadurch gekennzeichnet, dass** es als pflanzlichen Zusatz Kurkuma enthält.

3. Zahnreinigungsmittel nach einem der Ansprüche 1 oder 2, **dadurch gekennzeichnet, dass** der pflanzliche Zusatz als Pulver und/oder Essenz und/oder Paste vorliegt.

4. Zahnreinigungsmittel nach einem der vorhergehenden Ansprüche, **dadurch gekennzeichnet, dass** der pflanzliche Zusatz gewonnen wird, indem aus der jeweiligen Pflanze, insbesondere der Wurzel und/oder den Blättern entweder i) durch Trocknen und Pulverisieren des Pflanzenmaterials ein Pulver oder ii) durch Kochen in Wasser ein wässriger Sud oder iii) durch Einlegen in Alkohol ein alkoholischer Auszug oder iiii) durch Verarbeitung des fein zerriebenen Pflanzenmaterials mit mehr oder minder viskosen Flüssigkeiten eine Paste gewonnen wird.

5. Zahnreinigungsmittel nach einem der vorhergehenden Ansprüche, **dadurch gekennzeichnet, dass** es als Putzkörper Siliciumoxid, Calciumphosphat, Kaolin, Natriumpolyphosphat und/oder Kreide enthält.

6. Zahnreinigungsmittel nach einem der vorhergehenden Ansprüche, **dadurch gekennzeichnet, dass** es als Feuchthaltemittel Glycerin, Sorbitol und/oder Mannit enthält.

7. Zahnreinigungsmittel nach einem der vorhergehenden Ansprüche, **dadurch gekennzeichnet, dass** es Zusatzstoffe, vorzugsweise eine Fluoridquelle, insbesondere in Form von Flussspat, Natriumfluorid, Natriummonoflurphosphat und/oder Kaliumfluorid, Tenside und/oder Aromastoffe enthält.

8. Zahnreinigungsmittel nach einem der vorhergehenden Ansprüche, **dadurch gekennzeichnet, dass** es Natriumhydrogencarbonat aufweist.

9. Zahnreinigungsmittel nach einem der vorhergehenden Ansprüche, **dadurch gekennzeichnet, dass** es Xanthan Gum aufweist.

10. Zahnreinigungsmittel nach einem der vorhergehenden Ansprüche, **dadurch gekennzeichnet, dass** es Titanoxid aufweist.

11. Zahnreinigungsmittel nach einem der vorhergehenden Ansprüche, **dadurch gekennzeichnet, dass** es einen Süßstoff, insbesondere Saccharin aufweist.

12. Zahnreinigungsmittel nach einem der vorhergehenden Ansprüche, **dadurch gekennzeichnet, dass** es Zinkchlorid und/oder Methylparaben aufweist.

13. Zahnreinigungsmittel nach einem der vorhergehenden Ansprüche, **dadurch gekennzeichnet, dass** es 20 bis 40 Gew.-% Wasser, 10 bis 20 Gew.-% Putzkörper, 10 bis 45 Gew.-% Feuchthaltemittel, ca. 10 Gew.-% Zusatzstoffe und 0,1 bis 20 Gew.-% pflanzlichen Zusatz und/oder Propolis enthält.
